# EUROPEAN PATENT APPLICATION

(11) **EP 2 474 360 A2**
(43) Date of publication of application: **11.07.2012**
(21) Application number: 12150270.2
(22) Date of filing: 05.01.2012
(51) Int. Cl.: B01L 3/00

(54) **Microfluidic device and analyte detection method using the same**

(30) Priority: 10.01.2011 KR 20110002168
(71) Applicant: Samsung Electronics Co., Ltd., Suwon-si, Gyeonggi-do 443-742 (KR)
(72) Inventor: Kim, Kui Hyun, Gyeonggi-do (KR); Lee, Beom Seok, Gyeonggi-do (KR)
(74) Representative: Hylarides, Paul Jacques

(57) **Abstract**

Provided is a micro-fluidic device having multiple reaction chambers to simultaneously detect a plurality of different analytes, and an analyte detection method using the same. The micro-fluidic device includes multiple reaction chambers containing a plurality of capture materials to be combined with different analytes, multiple channels connecting the multiple reaction chambers, and valves provided within the multiple channels to control fluid flowing through the channels.

## Description

### BACKGROUND

### 1. Field

Apparatuses and methods consistent with exemplary embodiments relate to a micro-fluidic device and an analyte detection method using the same, and more particularly, to a micro-fluidic device for detecting a plurality of different analytes in a single micro-fluidic device, as well as an analyte detection method using the above micro-fluidic device.

### 2. Description of the Related Art

A micro-fluidic device refers to an apparatus used for executing biological or chemical reactions using a small amount of fluid.

In general, a micro-fluidic structure of a micro-fluidic device, which has at least one independent function, typically includes a chamber adapted for containing a fluid therein, and a channel through which the fluid may flow. As is known in the art, a device having a micro-fluidic structure mounted on a substrate in a chip form, such that some experiments involving biological and/or chemical reactions can be conducted on a small chip, in order to execute several experimental processes and/or operations on the structure, is referred to as a 'Lab-on-a-chip'. In order to move a fluid within the micro-fluidic structure, drive pressure is generally required. As such, drive pressure, capillary pressure or pressure generated using an additional pump may be used. In recent years, disc-type micro-fluidic devices having a micro-fluidic structure mounted on a rotational platform, which uses centrifugal force to move a fluid and/or execute a series of tasks have been proposed.

Such devices typically include a space wherein a variety of reagents for analysis of an analyte are stored, as well as a space wherein a reaction of the analyte and the reagents occurs. Because space within a micro-fluidic device is limited, in cases in which a plurality of analytes will be assessed in a single micro-fluidic device, there is little room for reaction space. Although increasing the size of the micro-fluidic device may enlarge the analysis space, doing so increases the cost of manufacturing the micro-fluidic device, and therefore renders it ineffective. Accordingly, providing the ability to assess a plurality of analytes without altering the size of the micro-fluidic device is desirable.

### SUMMARY

Exemplary embodiments provide a micro-fluidic device having multiple reaction chambers to simultaneously detect a plurality of different analytes in a single micro-fluidic device, as well as a test method of the analyte using the same.

According to an aspect of an exemplary embodiment, there is provided a micro-fluidic device including at least one micro-fluidic structure, which includes: multiple reaction chambers containing a plurality of capture materials, each of which will be combined with different analytes; multiple channels for connecting the multiple reaction chambers; and valves located on the multiple channels to control fluid flow through the channels.

The channels are placed between the multiple reaction chambers and may provide fluid communication of two or more of the multiple reaction chambers.

Each valve may be an open valve, thereby allowing the fluid to flow prior to application of power.

The valve may be a mixture including a phase transition material and a heat emitting fluid.

The phase transition material may be selected from the group consisting of wax, gel and thermoplastic resins.

The heat emitting fluid may include a carrier oil and a plurality of micro-heating particles dispersed in the carrier oil. In an exemplary embodiment, the micro-heating particle may be selected from the group consisting of micro-metal oxides, polymer particles, quantum dots and magnetic beads.

The micro-fluidic device may further include an external energy source to supply energy to the valve.

The external energy source may be a laser light source.

The capture material may include, but is not limited to, any one or more material selected from the group consisting of antibodies, antigens, receptors, ligands, oligo-nucleotides, haptens and aptamers.

The capture material may be combined with any one or more of polystyrene beads or plates.

The reaction chamber may include a fixed region in which the capture material is secured and placed.

The fixed region may be a substrate comprising a material selected from the group consisting of polymethylmethacrylate (PMMA), cyclic olefin copolymers (COC), polystyrene and polycarbonate (PC).

The micro-fluidic structure may further include: a first buffer chamber containing a plurality of binders (commonly referred to as 'conjugates') to be combined with different analytes; a second buffer chamber containing a substrate material (e.g., a coloring agent) to react with the conjugate; and a third buffer chamber containing a stop solution to stop the substrate reaction.

The conjugate may be any one selected from the group consisting of antibodies, antigens, receptors, ligands, oligo-nucleotides, haptens and aptamers.

According to an aspect of another exemplary embodiment, there is provided an analyte detection method including: preparing a composite by transporting a sample containing a plurality of analytes and a conjugate to multiple reaction chambers, and combining the same with capture materials contained in the multiple reaction chambers. After forming the composite, the channels that connect the multiple reaction chambers are closed. After closing the channels, a substrate material is transported to the multiple reaction chambers to enable reaction thereof with the composite. Light and/or color variation generated by reaction between the substrate material and the composite to calculate a concentration of each of the plurality of analytes contained in the sample is then measured.

Preparing a composite by transporting the sample and the conjugate to multiple reaction chambers for combining with capture materials may include: introducing the sample into the micro-fluidic device and transporting the sample to the multiple reaction chambers. Thereafter, when the sample is fed into the multiple reaction chambers, it is combined with the capture materials contained in the multiple reaction chambers to prepare first composites. After the first composites are prepared, a conjugate may be transported to the multiple reaction chambers, thereby combining the conjugate with each of the first composites to prepare a second composite.

The preparation of the first composite may include combining a plurality of different analytes contained in the sample with different capture materials contained in the multiple reaction chambers to form a plurality of different first composites.

After the any composite is formed, a washing buffer may be introduced into the multiple reaction chambers to remove any residue which does not form the composite.

The closing of the channels connecting the multiple reaction chambers may include applying energy to a valve mounted on each of the channels to melt constituents of the valve, thereby closing the channel.

The transportation of the substrate material to the multiple reaction chambers may include introducing the substrate material into each of the multiple reaction chambers, thus enabling reaction thereof with the conjugate as a constituent of the composite.

The method may further include introducing a stop solution into each of the multiple reaction chambers to stop the reaction between the substrate material and the composite.

### BRIEF DESCRIPTION OF THE DRAWINGS

The above and/or other aspects will become apparent and more readily appreciated from the following description of exemplary embodiments, taken in conjunction with the accompanying drawings of which:
FIG. 1 is a schematic view illustrating the structure of a valve according to an exemplary embodiment;
FIGS. 2A to 3B are cross-sectional views illustrating the structure of a valve according to an exemplary embodiment; and
FIG. 4 is a flow diagram illustrating a test process of an analyte using a micro-fluidic device according to an exemplary embodiment.

### DETAILED DESCRIPTION

Hereinafter, exemplary embodiments will be described with reference to the accompanying drawings. However, the present disclosure may be embodied in various other forms, which are not particularly restricted to those described herein.

In the accompanying drawings, like reference numerals denote elements substantially having the same configuration or performing similar functions and actions throughout the drawings. Separate structures such as a chamber, a channel, and the like are simply illustrated, and dimensional ratios of the same may be different from actual scales thereof. In expressions such as 'micro-fluidic device,' 'microparticle,' etc., the term 'micro' should not be construed as a unit of size, but rather, is used in contrast with the term 'macro'.

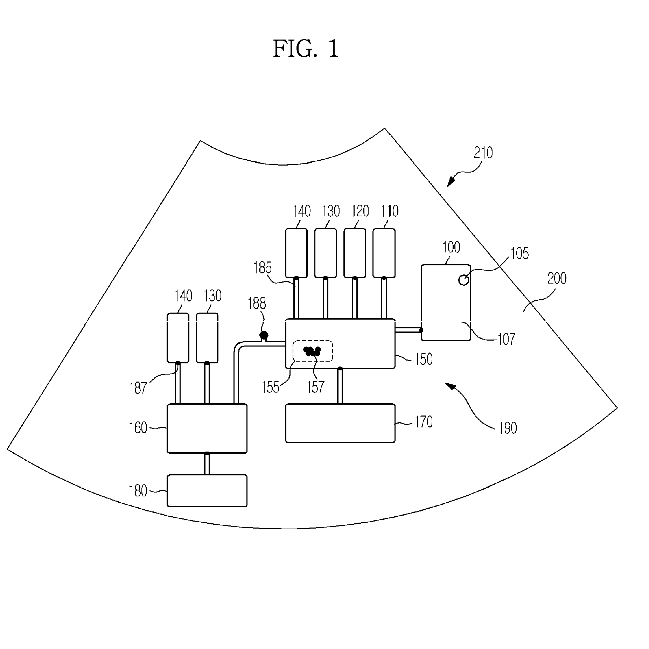

FIG. 1 is a schematic view illustrating the configuration of a micro-fluidic device according to one embodiment.

As illustrated in FIG. 1, a micro-fluidic device 210 according to an embodiment of the present invention may include at least one micro-fluidic structure 190, which includes a platform 200, a plurality of chambers (100, 110, 120, 130, 140, 150, 160, 170, and 180) provided on the platform 200, at least one channel 185 through which the chambers are connected, and at least one valve 187 (denoted by black circles) for opening and closing the channel, as well as a detection unit (not shown).

The platform 200 may include, but is not limited to, a circular disc-type platform. The platform may be formed using acrylic or other plastic materials, each of which is easily formable and has a biologically inactive surface. However, without particular limitation thereto, any materials having chemical or biological stability, optical transparency, and/or mechanical workability may be sufficiently used. That is, the platform may be fabricated using at least one material selected from the group consisting of plastic, polymethylmethacrylate (PMMA), glass, mica, silica, a silicon wafer material, plastics, and the like. In an exemplary embodiment, the plastic material is selected in view of economic merits and simple workability. Thus, exemplary commonly available plastic materials may include, but are not limited to, polypropylene, polyacrylate, polyvinylalcohol, polyethylene, polymethylmethacrylate, polycarbonate, etc.

In one exemplary embodiment, the platform may include multiple layers of plates. For example, a depressed intaglio structure corresponding to a chamber or a channel may be formed on a side at which two plates face each other. Thus, when two or more intaglio structures on opposing plates are combined, an empty space and/or channel may be formed inside the platform. The combining of such plates may be achieved using an adhesive, two-sided adhesive tape, ultrasonic welding, etc.

One or a plurality of micro-fluidic structures 190 may be provided on the platform. For instance, after partitioning the platform into several sections, individual micro-fluidic structures 190 may be placed independently of one another within each of the sections, as appropriate.

The term 'micro-fluidic structure' used herein refers to a general structure which includes a plurality of chambers, a plurality of channels and a plurality of valves, and induces a fluid flow. Therefore, the 'micro-fluidic structure' may form a specific unit with one or more different functions according to the arrangement of the chambers, the channels, the valves, and/or the kinds of materials received within the structure.

In an exemplary embodiment, the platform may be a rotatable disc type platform and centrifugal force may be used as drive pressure to transport a fluid. However, the platform is not particularly limited to such a disc type and may adopt any circular disc shape or a rotatable sector shape that is placed and fixed on a rotatable frame. In order to turn the platform, a rotational driving part (not shown) may be included to allow high speed rotation of the platform.

The micro-fluidic structure 190 may include a sample chamber 100, buffer chambers 110, 130 and 140, a washing buffer chamber 120, reaction chambers 150 and 160, and detection chambers 170 and 180.

The sample chamber 100 may provide a space in which a fluid sample such as blood is received.

The sample chamber 100 may have a sample inlet 105 through which the sample is injected and a sample receiving part 107. The receiving part may have an outlet connected to the reaction chamber 150 by a channel 185, and may further have a valve 187 mounted on the outlet to control flow of the fluid sample through the channel 185. The valve 187 may be any one selected from different types of micro-fluidic valves. For instance, the valve 187 may be a so-called 'normally closed valve,' wherein the valve is closes the channel to prevent a fluid from flowing unless the valve is opened due to the supply of external power. In an exemplary embodiment, the sample chamber 100 may further include a capillary valve structure through which the sample passes only when a predetermined pressure is applied. When provided between the sample inlet 105 and sample receiving part 107, the capillary valve structure allows the injected sample to flow from the sample inlet 105 toward the sample receiving part 107 by injection pressure while preventing the fluid sample from flowing backward to the sample inlet.

The sample chamber 100 may receive a fluid such as blood, and may further include a sample separation part as a structure for centrifuging the fluid into a supernatant (i.e., serum, plasma, etc.) and a precipitate (i.e., blood cells). The sample separation part for centrifugation of the fluid sample may have a variety of morphologies. The sample separation part may include a supernatant collection part (not shown) and a precipitate collection part (not shown) located at the end of the supernatant collection part to collect the precipitate having relatively high specific gravity. The supernatant collection part may have a channel to distribute the centrifuged supernatant into the reaction chamber. The valve may control flow of the sample passing through the channel. The valve may adopt any micro-fluidic valve in various shapes. For instance, the valve may be a so-called 'normally closed valve,' which closes the channel to block fluid flow until the valve receives external power and is opened, as described above.

A supernatant metering chamber to determine an amount of the supernatant may be provided between the sample separation part and the reaction chamber 100. The supernatant metering chamber may have a volume to receive a desired amount of supernatant for examination. At the outlet of the supernatant metering chamber, a valve may be provided to control fluid flow. The valve, as described above, may be a so-called 'normally closed valve.' The supernatant metering chamber may be connected to the reaction chamber 100 through a channel 185. Although not illustrated in the drawing, a chamber and a channel to receive excess fluid sample remaining after metering may be further provided between the sample separation part and the supernatant metering chamber.

The buffer chambers 110, 130 and 140 may contain a reaction solution required for examination (analysis) of an analyte.

In an exemplary embodiment, the first buffer chamber 110 may contain a first buffer. The first buffer may be a conjugate buffer to combine with an analyte through antigen-antibody reaction, ligand-receptor bonding, etc. The conjugate may be include a binder to be combined with the analyte and a label to detect the analyte. The binder may be a capture probe that binds to the analyte, which includes antigens, antibodies, receptors, ligands, oligo-nucleotides, haptens, aptamers, etc., and may be selected depending upon types of analytes being tested. The label may include, but is not limited to, metal colloids such as latex beads, gold colloids, silver colloids, etc.; enzymes (HRP, ALP, etc.); color materials; fluorescent materials or nanoparticles containing the same; phosphorescent materials or nanoparticles containing the same; nocti-luminescent materials; light emitting materials; pigment-containing liposomes; metal nanoparticles; carbon nanoparticles; colored polymeric nanoparticles; super para-magnetic materials or nanoparticles containing the same; lanthanide (III) chelates or nanoparticles containing the same; radioactive isotopes, or the like.

In an exemplary embodiment, the first buffer chamber 110 may receive a plurality of conjugates, each of which specifically binds with one or more of a plurality of different analytes. For example, two types of conjugates that specifically bind to a prostate specific antigen (PSA) and testosterone, respectively, may be received therein. In another example, two alternative types of conjugates that specifically bind to thyroid stimulating hormone (TSH) and free T4 protein (fT4), respectively, may be received therein for detection of thyroid gland diseases. In another example, four types of conjugates that specifically bind to the afore-mentioned four different materials may be received therein. The foregoing is listed for illustrative purpose only and does not particularly limit the present invention.

A valve 187 may be positioned at the outlet of the first buffer chamber 110. The valve may be a closed valve, as described above. A first buffer may then be introduced into the first buffer chamber 110 and the valve maintains the buffer in a sealed condition until the valve is opened. The first buffer chamber may be connected to a first metering chamber (not shown) to supply a predetermined amount of the first buffer required for examination to the reaction chamber. The first metering chamber may be connected to the first buffer chamber 110 through a valve 187, and another valve 187 may be provided at the outlet of the first metering chamber. Either or both of the valves may be a normally closed valve, as described above. By opening the valve, a predetermined amount of the first buffer, metered by the first metering chamber, may be supplied to the reaction chamber.

A second buffer chamber 130 may contain a second buffer. In an exemplary embodiment, the second buffer may receive a substrate material such as 3,3',5,5'-tetramethylbenzidine (TMB), which generates a specific color upon reaction with a product resulting from conjugation or a competitive reaction. A valve 187 may be provided at the output of the second buffer chamber 130, and the valve may be a normally closed valve, as described above, to maintain the same in a sealed condition until the valve is opened. The second buffer chamber 130 may be connected to a second metering chamber (not shown) to supply a predetermined amount of the second buffer to the reaction chamber. The second metering chamber may be connected to the second buffer chamber 130 through a valve 187, while another valve 187 may be provided at the outlet of the second metering chamber. The valve may be a normally closed valve, as described above. By opening the valve, a predetermined amount of the second buffer, metered by the second metering chamber, may be supplied to the reaction chamber 150. Referring to FIG. 1, in an exemplary embodiment, the second buffer chamber 130 is connected to a first reaction chamber 150, with a separate second buffer chamber 130 begin connected to a second reaction chamber 160. In another exemplary embodiment, a quantified substrate material is supplied through the second metering chamber (not shown) from the second buffer chamber 130, and may be delivered to the first reaction chamber 150 through a channel 185 connected to the first reaction chamber 150, and then subsequently, through a channel 185 to the second reaction chamber 160.

A third buffer chamber 140 may contain a third buffer. In an exemplary embodiment, the third buffer is a stop solution to stop the substrate reaction. A valve 187 may be provided at the output of the third buffer chamber 140, and the valve may be a normally closed valve, as described above, to maintain the same in a sealed condition until the valve is opened. The third buffer chamber 140 may be connected to a third metering chamber (not shown) to supply a predetermined amount of the third buffer to the reaction chamber 150. The third metering chamber may be connected to the third buffer chamber 140 through a valve 187, while another valve 187 may be provided at the outlet of the third metering chamber. The valve may be a normally closed valve as described above. By opening the valve, a predetermined amount of the third buffer metered by the third metering chamber may be supplied to the reaction chamber 150. Referring to FIG. 1, in an exemplary embodiment, the third buffer chamber 140 is connected to the first reaction chamber 150, and a separate third buffer chamber 140 may be connected to the second reaction chamber 160, respectively, however, this is only an illustrative example. In another exemplary embodiment, a quantified substrate material is supplied through the third metering chamber (not shown) from the third buffer chamber 140, and may be delivered to the first reaction chamber 150 through a channel 185 connected to the first reaction chamber 150, and then subsequently, through a channel 185 connected to the second reaction chamber 160.

The washing buffer chamber 120 may contain a washing buffer to clean residue after reaction of analytes and buffers. The washing buffer chamber 120 may be connected to the reaction chamber 150 through a valve 187. The valve 187 may be a normally closed valve, as described above to maintain the same in a sealed condition until the valve is opened.

In an exemplary embodiment, the reaction chamber 150 may receive a capture material, which binds with an analyte contained in a liquid sample or supernatant through an antigen-antibody reaction, ligand-receptor bonding, etc. The capture material is a capture probe for binding analytes, and may include antibodies, antigens, receptors, ligands, oligo-nucleotides, haptens or aptamers, etc., which are selected depending upon analyte type. For instance, in the case where an analyte is a carbamate insecticide, the capture material may be acetylcholine esterase (AChE). In another example, if the analyte is an antigen, the capture material may be a capture antibody.

The reaction chamber may have a fixed region in which the capture material is secured and placed. Exemplary materials for forming the fixed region include, but are not limited to, polymethyl methacrylate (PMMA), a cyclic olefin copolymer (COC), polystyrene and polycarbonate (PC). In one exemplary embodiment, the capture material may be received in the reaction chamber 150, while being adhered to polystyrene beads 157 or plates.

The micro-fluidic structure 190 according to another exemplary embodiment may include multiple reaction chambers (100, 160) to receive a capture material capable of specifically binding with respective analytes, in order to simultaneously detect a plurality of different analytes. FIG. 1 shows two reaction chambers, that is, a first reaction chamber 150 and a second reaction chamber 160, however, this is only an illustrative example. Thus, the micro-fluidic structure may include two or more reaction chambers. For example, a first reaction chamber may contain a capture material that specifically binds with a prostate specific antigen (PSA) for examination of prostate cancer, while a second reaction chamber may include a capture material that specifically binds with testosterone. In another exemplary embodiment, a first reaction chamber may receive a capture material that specifically binds with thyroid stimulating hormone (TSH) for examination of thyroid gland diseases, while a second reaction chamber may receive a capture material that specifically binds with free T4 protein (fT4). In another exemplary embodiment, the micro-fluidic structure may include four reaction chambers, each of which containing capture materials that specifically bind to the foregoing four materials, respectively. The foregoing is only described as illustrative examples without being particularly limited thereto.

As such, through multiple reaction chambers containing capture material specifically bound to different analytes, a plurality of different analytes may be simultaneously detected within the respective reaction chambers. Hereinafter, for illustrative purposes only, the first reaction chamber 150 is used to detect prostate specific antigen (PSA) and the second reaction chamber 160 is used to detect testosterone, in order to explain structural characteristics of the plurality of reaction chambers.

The plurality of reaction chambers may be connected through channels 185 which are positioned between the reaction chambers and provide fluid communication, controlled by one or more valves 187, between any of the reaction chambers. Any one or more of the valves 187 may be a so-called normally open valve, which as discussed above, refers to a valve that closes upon receiving external energy or power, but is open before supplying power, thus allowing the fluid flowing therethrough. FIG. 1 illustrates a normally open valve 188, which is present at the channel 185 between the first (150) and second (160) reaction chambers. The normally open valve 188 is shown as a large black circle, while normally closed valves 187 are indicated as small black circles.

Accordingly, multiple reaction chambers are fluidly connected until the channels 185 are closed. Since the multiple reaction chambers are fluidly connected, any one or more of the fluid sample or supernatant, a conjugate buffer and a washing buffer may be delivered to any one of the reaction chambers, and may subsequently be transported to any other reaction chambers through the channels 185, as appropriate. As such, any material that is present in a single reaction chamber may be transported to any other reaction chamber, as necessary. Accordingly, instead of increasing the number of the sample chambers 100, it may be sufficient to increase the number of reaction chambers only corresponding to the number of analytes being tested, thereby simultaneously detecting a plurality of different analytes within a limited space.

After the steps required for detection of an analyte in each reaction chamber, as described above (i.e., delivery of the foregoing supernatant, conjugate and washing buffer to the reaction chamber) are completed, the open valve 188 may be closed and the substrate material is transported from the second buffer chamber 130 connected to each of the reaction chambers, respectively. In one exemplary embodiment, the substrate material transported to each of the reaction chambers may react with the conjugate.

The detection chambers 170 and 180 are in fluid communication with each of the multiple reaction chambers through channels 185, and may receive a final fluid for detection from the reaction chambers after completing all reactions. Detection of an analyte may be conducted in the detection chambers 170 and 180 or, otherwise, the analyte may be directly detected in the reaction chambers after all reactions are completed. However, mechanical structures (e.g., beads 157) to which the capture material may be attached in the reaction chamber may reflect irradiated light for detecting the analyte, thereby making detection difficult. Therefore, when such is the case, detection of the analyte may be performed in the detection chambers 170 and 180, each of which receives the final fluid from the reaction chambers after completing the respective reactions.

The detection part (not shown) may be provided outside the micro-fluidic structure 190, and sense optical properties such as fluorescence, light emission and/or light absorption. In an exemplary embodiment, the detection part may include a light source, a light reception part which is arranged to correspond to the light source and receive light passing through the detection chambers 170 and 180, and an analysis part to analyze the optical properties of the light received by the light reception part and then calculate the concentration of an analyte.

The light source may comprise a light source flashing at a predetermined frequency, a semiconductor light emitting device such as a light emitting diode (LED), a laser diode (LD), a gas discharge lamp such as a halogen lamp, a xenon lamp, etc. The light reception part may generate electrical signals depending upon the intensity of incident light and may include, for example, a depletion layer photo-diode, an avalanche photo-diode (APD), photomultiplier tubes (PMT), or the like.

The light source and the light reception part of the detection part according to one exemplary embodiment o may be arranged opposite one another with the micro-fluidic structure 190 interposed therebetween. In addition, a light route may be guided through a mirror or a light guide component. The analysis part may calculate the concentration of the analyte using a standard curve stored therein.

The afore-mentioned normally closed valve 187 as well as a normally open valve 188 will be described in more detail, as follows. The open valve and the closed valve each actively react to external power or energy.

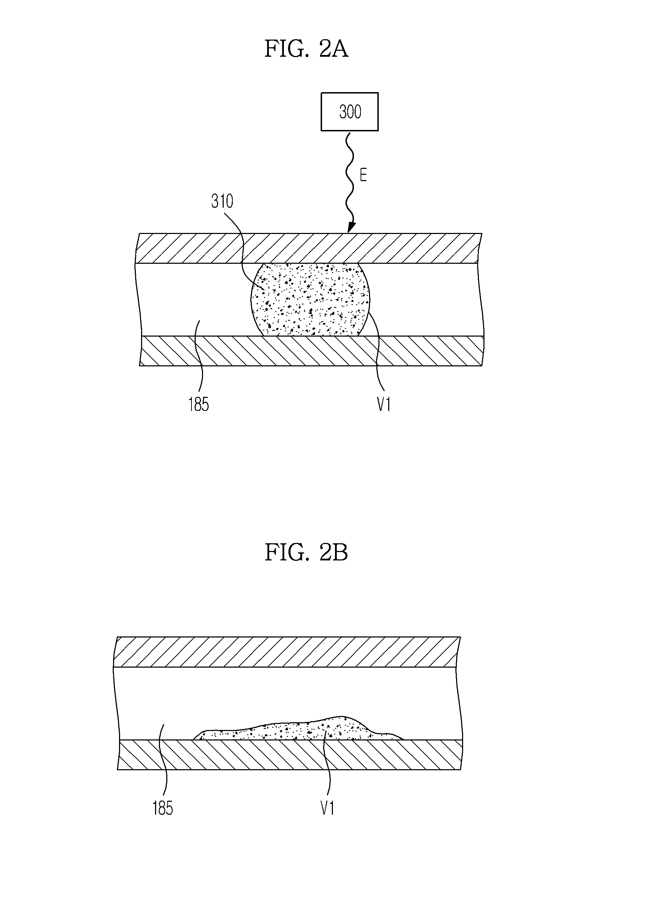

FIGS. 2A and 2B are cross-sectional views illustrating exemplary embodiments of the normally closed valve 187 adapted for use in the micro-fluidic structure 190. The closed valve 187 may contain a valve material V1 which is solid at room temperature. The valve material V1 is present in a solidified condition in a channel 185 and blocks the channel 185, as shown in FIG. 2A. The valve material V1 may be melted at a high temperature, and moved to a space inside the channel 185, as shown in FIG. 2B, and may be solidified again while opening the channel 185. Externally irradiated energy E may be electromagnetic waves, and an energy source may be a laser light source to irradiate a laser, a light emitting diode to irradiate visible light or infrared light, or a xenon lamp. As the laser light source, at least one laser diode is included. The external energy source 300 may be suitably selected on the basis of wavelengths of the electromagnetic waves which may be absorbed by light emitting particles contained in the valve material V1. Such a valve material V1 may comprise thermoplastic resin. In one exemplary embodiment, the valve material V1 may include a phase transition material which is solid at room temperature, such as wax. The wax may be solid at room temperature and become liquid when it is heated. Exemplary waxes include, but are not limited to, paraffin wax, microcrystalline wax, synthetic wax, natural wax, and so forth. The phase transition material may be a gel or thermoplastic resin. In the case of a gel, polyacrylamide, polyacrylate, polymethacrylate, polyvinyl amide, etc. may be employed. In another exemplary embodiment, the valve material V1 may include numerous micro-heating particles 310 dispersed therein to absorb electromagnetic waves and then generate heat. The micro-heating particles may have a diameter of about 1 nm to about 100µm, in order to freely pass through a micro-channel 185 having a width of about 0.1 mm to about 1 mm. The micro-heating particles 310 may have a thermal property wherein the temperature thereof is rapidly increased when exposed to electromagnetic energy, for example, by a laser beam, thereby generating heat. Moreover, the micro-heating particles 310 may be uniformly dispersed in the wax. In order to exhibit the foregoing properties, each of the micro-heating particles 310 may have a core containing metal components and a hydrophobic surface structure. For instance, the micro-heating particle 310 may have a Fe core and a molecular structure including a plurality of surfactants which surround the Fe. In another exemplary embodiment, the micro-heating particles 310 may be stored in a dispersion state in a carrier oil. The carrier oil may also be hydrophobic to allow the micro-heating particles 310 having the hydrophobic surface structure to be uniformly dispersed in the carrier oil. Thus, a channel 185 may be closed by forming a uniform dispersion of the melted transition material and the carrier oil containing the micro-heating particles 310, and introducing the mixture into the channel 185.

Micro-heating particles 310 are not particularly limited to polymer particles, as provided herein for illustrative purposes. In another exemplary embodiment, the micro-heating particles 310 may be in a quantum dot or magnetic bead form. In another exemplary embodiment, the micro-heating particles may be micro-metal oxides such as A1203, Ti02, Ta203, Fe203, Fe304 or Hf02.

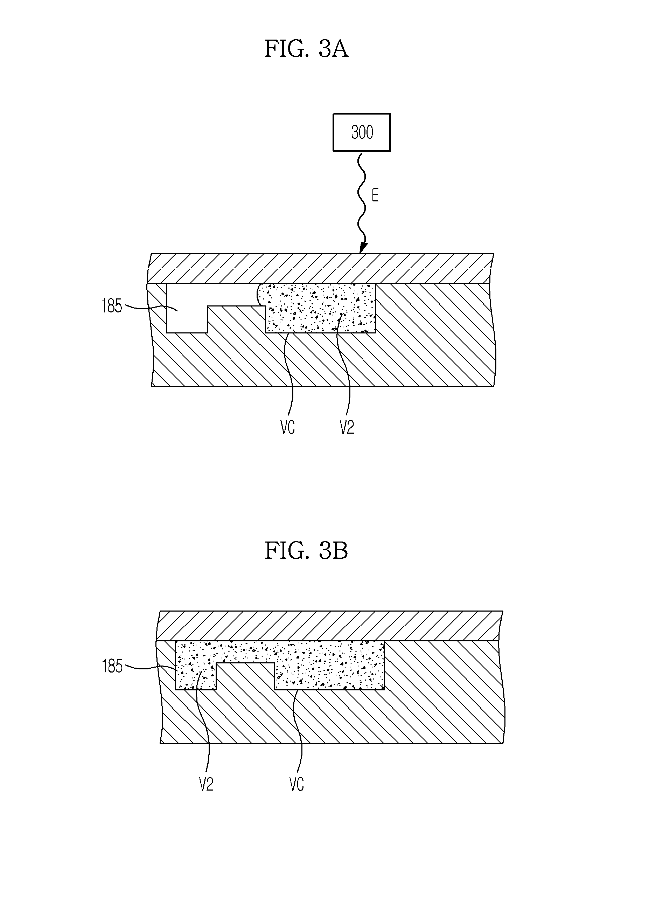

In another exemplary embodiment, the normally open valve 188 may include a phase transition material with or without micro-heating particles. FIGS. 3A and 3B are cross-sectional views illustrating one example of the normally open valve 188. The open valve 188 may a valve chamber VC connected to a part of the channel 185, and a valve material V2 contained in the valve chamber VC. The valve material V2 may be the same material as that of valve material V1 of the normally closed valve 187. As shown in FIG. 3A, the valve material V2 is present in the valve chamber VC prior to application of external energy, and therefore the channel 185 is open. Then, when external energy E is applied to the valve material V2, the valve material V2 melts, expands and flows into the channel 185. Upon entering channel 185, valve material V2 solidifies to block fluid flow through the channel 185. Referring to FIG. 1, the normally closed valve 187 is indicated as a small black circle while the normally open valve 188 is represented by a large black circle.

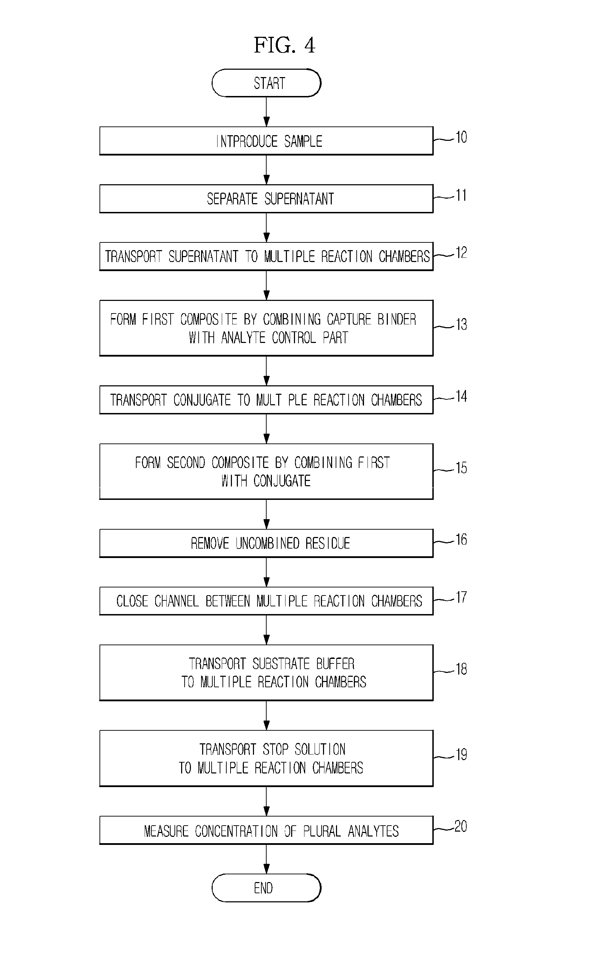

FIG. 4 is a flow diagram showing a method of detecting an analyte using the micro-fluidic device 210 according to one exemplary embodiment.

A fluid sample, such as whole blood collected from a subject, is introduced into a sample chamber 100 (operation 10) and, through centrifugation in a sample separation part, a supernatant containing serum or plasma is separated from a precipitate containing blood cells (operation 11).

After separation of the supernatant, a normally closed valve 187 of a channel connecting the sample separation part and a first reaction chamber 150 is opened and the supernatant is transported to the first reaction chamber 150 using centrifugal force generated by rotation of the platform 200 as drive pressure (operation 12). Here, the supernatant may also be delivered to the second reaction chamber 160 through the channel connecting the first reaction chamber 150 and the second reaction chamber 160.

When the supernatant flows into the first and second reaction chambers, the analyte being tested, which is present in the supernatant, binds to the capture materials contained in the first and second reaction chambers. Such combination of the analyte and the capture material may provide a first composite (operation 13). In this regard, in order to facilitate combination of the analyte and the capture material, the platform 200 may be shaken several times from side to side.

After forming the first composite, a closed valve 187 of a channel 185 connecting the first buffer chamber 110 and the first reaction chamber 150 is opened, and a conjugate buffer is transported to the first reaction chamber 150 using centrifugal force generated by rotation of a platform 200 as drive pressure (operation 14). Here, the conjugate buffer transported to the first reaction chamber 150 may also be delivered to the second reaction chamber 160 through a channel 185 connecting the first reaction chamber 150 and the second reaction chamber 160.

When the conjugate buffer flows into the first and second reaction chambers, the conjugate buffer binds with the respective first composites contained in each of the first and second reaction chambers. In one exemplary embodiment, the conjugate buffer may be combined with the analyte contained in the first composite, to form a second composite having a sandwich structure (operation 15). In this regard, in order to facilitate combination of the conjugate and the first composite, the platform 200 may be shaken several times from side to side.

After forming the second composite, a closed valve of a channel 185 connecting the washing buffer chamber 120 and the first reaction chamber 150 is opened, and the washing buffer is transported to the first reaction chamber 150 using centrifugal force generated by rotation of a platform 200 as drive pressure. Here, the washing buffer transported to the first reaction chamber 150 may also be delivered to the second reaction chamber 160 through the channel connecting the first reaction chamber 150 and the second reaction chamber 160. The washing buffer transported to the first and second reaction chambers may remove residues that do not form the second composite, but remain in the reaction chambers (operation 16).

After removal of such unbound residues, the valve of the channel connecting the first and second reaction chambers is closed (operation 17). Thus, the valve of the channel between the first and second reaction chambers may be a normally open valve, as described above.

In the case where the channel between the first and second reaction chambers is closed, both the closed valve of the channel connecting the second buffer chamber 130 and the first reaction chamber 150, and the closed valve of the channel connecting the second buffer chamber 130 and the second reaction chamber 160, are opened and a substrate material is transported to the first and second reaction chambers 150 and 160 using centrifugal force generated by rotation of the platform 200 as drive pressure (operation 18). When the substrate material is transported to the first and second reaction chambers, the conjugate forming the second composite may react with the substrate material, thus inducing color variation and/or generating a specific color.

After a predetermined time, both the closed valve of the channel connecting the second buffer chamber 140 and the first reaction chamber 150, and the closed valve of the channel connecting the second buffer chamber 140 and the second reaction chamber 160 are opened to allow a stop solution to be transported to the first and second reaction chambers 150 and 160, using centrifugal force generated by rotation of the platform 200 as drive pressure (operation 19). When the stop solution enters the first and second reaction chambers, substrate reaction between the second composite and the substrate material may be terminated.

After completing the substrate reaction, the second composite formed in the first reaction chamber 150 and the second composite formed in the second reaction chamber 160 are each measure with regard to extent of coloration by the detection part, thereby measuring the concentration of each analyte (operation 20). In one exemplary embodiment, measurement of the concentration of the analyte may be performed directly in the first and second reaction chambers. In another exemplary embodiment, after delivering the washing buffer to the first and second reaction chambers, the second composite is separated from the solid support (such as beads), and transported to the first and second detection chambers 170 and 180, in order to measure the concentration of the analyte.

According to an aspect of the exemplary embodiments, several analytes may be simultaneously tested (or detected) in a single micro-fluidic device, thereby providing rapid and effective testing thereof while reducing the cost of manufacturing the device.

Moreover, a positive and/or negative control may be included in the micro-fluidic structure to confirm the quality of the test.

Although exemplary embodiments have been described above with reference to the accompanying drawings, it is clearly understood that these exemplary embodiments do not particularly restrict the scope of the inventive concept. Accordingly, it would be appreciated by those skilled in the art that various substitutions, variations and/or modifications may be made in these exemplary embodiments without departing from the principles and spirit of the inventive concept. Therefore, it is obviously understood that the inventive concept is not restricted to the technical configurations and arrangements illustrated above.

## Claims

1. A micro-fluidic device comprising:
a plurality of reaction chambers containing a plurality of capture materials to combine with different analytes, respectively;
a plurality of channels connecting the plurality of reaction chambers; and
a plurality of valves provided within the plurality of channels and configured to control fluid flowing through the channels.

2. The micro-fluidic device according to claim 1, wherein the channels are positioned to provide fluid communication between the plurality of reaction chambers.

3. The micro-fluidic device according to claim 1 or 2, wherein each valve is an open valve that is open to enable the fluid to flow prior to application of energy to the valve, comprises a mixture comprising a phase transition material and a heat emitting fluid, wherein the phase transition material is selected from the group consisting of wax, gel and thermoplastic resin.

4. The micro-fluidic device according to claim 3, wherein the heat emitting fluid comprises a carrier oil and a plurality of micro-heating particles dispersed in the carrier oil, and wherein the micro-heating particles are selected from the group consisting of micro-metal oxide, polymer particles, quantum dots and magnetic beads.

5. The micro-fluidic device according to any of the preceding claims, further comprising an external energy source to supply energy to the valves, wherein the external energy source comprises a laser beam source.

6. The micro-fluidic device according to any of the preceding claims, wherein the capture material is selected from the group consisting of antibodies, antigens, receptors, ligands, oligo-nucleotides, haptens and aptamers, wherein the capture material binds to a polystyrene bead or plate.

7. The micro-fluidic device according to any of the preceding claims, wherein at least one of the reaction chambers has a fixed region in which the capture material is secured and arranged, wherein the fixed region is a substrate consisting of a material selected from polymethylmethacrylate (PMMA), cyclic olefin copolymer (COC), polystyrene and polycarbonate (PC).

8. The micro-fluidic device according to any of the preceding claims, further comprising:
a first buffer chamber containing a plurality of conjugates that bind to different analytes;
a second buffer chamber containing a substrate material that reacts with the conjugate and generates color; and
a third buffer chamber containing a stop solution to terminate the reaction with the substrate material.

9. The micro-fluidic device according to claim 8, wherein the conjugate is any one selected from the group consisting of antibodies, antigens, receptors, ligands, oligo-nucleotides, haptens and aptamers.

10. An analyte detection method comprising:
transporting a sample containing a plurality of analytes, and a conjugate to a plurality of reaction chambers, forming a composite by combining the sample and conjugate with a capture material contained in the plurality of reaction chambers;
after the forming the composite, closing a channel connecting the plurality of reaction chambers;
after the closing the channel, transporting a substrate material to the plurality reaction chambers to allow reaction thereof with the composite; and
measuring light generated by reaction between the substrate material and the composite, to determine a concentration of the plurality of analytes in the sample.

11. The analyte detection method according to claim 10, wherein the forming the composite comprises:
introducing the sample into a micro-fluidic device to transport the sample to the plurality of reaction chambers;
after transporting the sample, allowing the plurality of analytes contained in the sample to bind to different capture materials contained in the plurality of reaction chambers, thereby forming a plurality of different first composites; and
after the forming the first composites, transporting the conjugate to the plurality of reaction chambers and allowing the conjugate to bind to the first composites, thereby forming second composites.

12. The analyte detection method according to claim 10 or 11, further comprising: after the forming the composite, introducing a washing buffer into each of the plurality of reaction chambers to remove residues which do not form the composite.

13. The analyte detection method according to claim 10, 11 or 12, wherein the closing the channel connecting the plurality of reaction chambers comprises applying energy to a valve of the channel to melt a valve material of the valve, thereby closing the channel.

14. The analyte detection method according to any of claims 10-13, wherein the transporting the substrate material to the multiple reaction chambers to react the same with the composite includes transporting the substrate material to the multiple reaction chambers, respectively, to execute substrate reaction thereof with the conjugate contained in the composite.

15. The method analyte detection according to any of claims 10-14, further comprising introducing a stop solution into each of the plurality of reaction chambers to stop the reaction of the substrate material with the composite.
